# EUROPEAN PATENT SPECIFICATION

(11) **EP 4 117 672 B1**
(45) Date of publication and mention of the grant of the patent: **20.05.2026**
(21) Application number: 21768328.3
(22) Date of filing: 12.03.2021
(51) Int. Cl.: A61K 31/65, A61P 31/12, A61P 31/14, C07C 237/00, C07C 237/24, C07C 237/26

(54) **CMT-3 FOR USE IN TREATING ILLNESSES CAUSED BY COVID-19**
CMT-3 ZUR BEHANDLUNG VON KRANKHEITEN, DIE DURCH COVID-19 VERURSACHT WERDEN
CMT-3 POUR LE TRAITEMENT DE MALADIES PROVOQUÉES PAR LA COVID-19

(30) Priority: 13.03.2020 US 202062989341 P
(43) Date of publication of application: 18.01.2023
(73) Proprietor: CMTX Biotech Inc., Kings Park, NY 11754 (US)
(72) Inventor: SCADUTO, Joseph, NY 11754 (US); KEANE, James, NY 11795 (US)
(74) Representative: V.O.
(86) International application number: PCT/US2021/022156
(87) International publication number: WO 2021/183918

(56) References cited:
- WO-A1-2005/082860
- WO-A1-2009/128913
- WO-A1-2019/135003
- WO-A2-2007/050379
- KR-A- 20140 145 980
- US-A1- 2015 216 826
- US-A1- 2017 021 011
- HUSTON NICHOLAS C ET AL: "Comprehensive in vivo secondary structure of the SARS-CoV-2 genome reveals novel regulatory motifs and mechanisms", MOLECULAR CELL, ELSEVIER, AMSTERDAM, NL, vol. 81, no. 3, 1 January 2021 (2021-01-01), pages 584, XP086487162, ISSN: 1097-2765, [retrieved on 20210101], DOI: 10.1016/J.MOLCEL.2020.12.041
- XU JIABAO ET AL: "Systematic Comparison of Two Animal-to-Human Transmitted Human Coronaviruses: SARS-CoV-2 and SARS-CoV", VIRUSES, vol. 12, no. 2, 22 February 2020 (2020-02-22), pages 1 - 17, XP055853415, DOI: 10.3390/v12020244
- MARTINA BIANCHI ET AL: "Sars-CoV-2 Envelope and Membrane Proteins: Structural Differences Linked to Virus Characteristics?", BIOMED RESEARCH INTERNATIONAL, vol. 2020, 30 May 2020 (2020-05-30), pages 1 - 6, XP055757449, ISSN: 2314-6133, DOI: 10.1155/2020/4389089
- YANG JINN-MOON ET AL: "Combinatorial Computational Approaches to Identify Tetracycline Derivatives as Flavivirus Inhibitors", PLOS ONE, vol. 2, no. 5, 9 May 2007 (2007-05-09), US, pages e428, XP093311529, ISSN: 1932-6203, DOI: 10.1371/journal.pone.0000428
- CHAN JASPER FUK-WOO ET AL: "Altered host protease determinants for SARS-CoV-2 Omicron", SCIENCE ADVANCES, vol. 9, no. 3, 20 January 2023 (2023-01-20), US, pages eadd3867, XP093311489, ISSN: 2375-2548, Retrieved from the Internet <URL:https://www.science.org/doi/pdf/10.1126/sciadv.add3867> DOI: 10.1126/sciadv.add3867
- SHI GUOLI ET AL: "Omicron Spike confers enhanced infectivity and interferon resistance to SARS-CoV-2 in human nasal tissue", NATURE COMMUNICATIONS, vol. 15, no. 1, 30 January 2024 (2024-01-30), UK, XP093311490, ISSN: 2041-1723, Retrieved from the Internet <URL:https://www.nature.com/articles/s41467-024-45075-8.pdf> DOI: 10.1038/s41467-024-45075-8
- WU CANRONG, LIU YANG, YANG YUEYING, ZHANG PENG, ZHONG WU, WANG YALI, WANG QIQI, XU YANG, LI MINGXUE, LI XINGZHOU, ZHENG MENGZHU, C: "Analysis of therapeutic targets for SARS-CoV-2 and discovery of potential drugs by computational methods", ACTA PHARMACEUTICA SINICA B, vol. 10, no. 5, 1 May 2020 (2020-05-01), pages 766 - 788, XP055776753, ISSN: 2211-3835, DOI: 10.1016/j.apsb.2020.02.008

## Description

### FIELD OF THE INVENTION

The methods of the present invention include the administration of a tetracycline compound to subjects, in need thereof, in an amount which is effective for the treatment of viruses that cause respiratory illness.

### BACKGROUND OF THE INVENTION

Coronaviruses (CoV) are a large family of viruses that cause illness ranging from the common cold to more severe diseases such as Middle East Respiratory Syndrome (MERS-CoV), Severe Acute Respiratory Syndrome (SARS-CoV) and Coronavirus Disease 2019 (COVID-19).

Coronaviruses are zoonotic, meaning they are transmitted between animals and people. Detailed investigations found that SARS-CoV was transmitted from civet cats to humans and MERS-CoV from dromedary camels to humans. Several known coronaviruses are circulating in animals that have not yet infected humans.

Coronavirus Disease 2019 (COVID-19) is an infectious disease caused by the Severe Acute Respiratory Syndrome Coronavirus 2 (SARS-CoV-2). The disease has spread globally since 2019, resulting in the 2019-20 coronavirus pandemic.

Common symptoms include fever, cough and shortness of breath. Muscle pain, sputum production and sore throat are some of the less common symptoms. While the majority of cases results in mild symptoms, some progress to pneumonia, severe acute respiratory syndrome, multi-organ failure and even death. The case fatality rate is estimated at between 1% and 5% but varies by age and other health conditions. The disease is more likely to occur in people whose immune system is impaired due to age, immunosuppressive therapy, psychological stress, or other factors.

The infection is spread from one person to others via respiratory droplets, often produced during coughing. Time from exposure to onset of symptoms is generally between two and 14 days, with an average of five days. The standard method of diagnosis is by reverse transcription polymerase chain reaction (rRT-PCR) from a nasopharyngeal swab or sputum sample. Antibody assays can also be used, using a blood serum sample. The infection can also be diagnosed from a combination of symptoms, risk factors and a chest CT scan showing features of pneumonia.

Recommended measures to prevent the disease include frequent hand washing, maintaining distance from other people, not touching one's face, covering mouth and nose when coughing and sneezing, and thoroughly cooking meat and eggs. The use of masks is recommended for those who suspect they have the virus, and their caregivers, but not for the general public.

Because a vaccine against SARS-CoV-2 is not expected to become available until 2021 at the earliest, a key part of managing the COVID-19 pandemic is trying to decrease the epidemic peak, known as flattening the epidemic curve. This helps decrease the risk of health services being overwhelmed and provides more time for a vaccine and treatment to be developed. There are no antiviral medications specific to COVID-19. Patients are managed with supportive care such as fluid and oxygen support

Accordingly, a need exists for an effective and safe method of preventing or inhibiting the symptoms associated with COVID-19.

### SUMMARY OF THE INVENTION

In one aspect, the present invention provides a non-antibacterial tetracycline compound in an amount that is effective to treat COVID-19 but has no antibacterial activity, for use in a method of treating COVID-19, in a subject in need thereof, wherein the non-antibacterial tetracycline compound is 6-demethyl-6-deoxy-4-de(dimethylamino)tetracycline (CMT-3), and wherein the method comprises the systemic administration of the non-antibacterial tetracycline compound.

In one embodiment, treatment is begun before the onset of COVID-19.

### DETAILED DESCRIPTION OF THE INVENTION

In one aspect, the present invention comprises the administration of a tetracycline compound to a subject, in need thereof, in an amount which is effective for the treatment of viral infections that cause respiratory illnesses, but which has substantially no antibacterial activity.

In one aspect, the present invention comprises the administration of a tetracycline compound to a subject, in need thereof, in an amount which is effective for the treatment of Coronavirus (CoV) infections, and illness caused by CoV, but which has substantially no antibacterial activity.

Examples of illnesses caused by CoV include Middle East Respiratory Syndrome (MERS-CoV), Severe Acute Respiratory Syndrome (SARS-CoV) and Coronavirus Disease 2019 (COVID-19).

Also described herein are methods that comprise the administration of a tetracycline compound to a subject, in need thereof, in an amount which is effective for the treatment of Swine Influenza and Avian Influenza, and illnesses caused by such, but which has substantially no antibacterial activity.

Swine influenza (also known as swine flu or pig flu) is a respiratory disease that occurs in pigs that is caused by the Influenza A virus. Influenza viruses that are normally found in swine are known as swine influenza viruses (SIVs). The known SIV strains include influenza C and the subtypes of influenza A known as H1N1, H1N2, H3N1, H3N2 and H2N3. H1N1: Influenza A virus subtype H1N1 (A/H1N1) is the subtype of influenza A virus that was the most common cause of human influenza (flu) in 2009, and is associated with the 1918 outbreak known as the Spanish flu.

Avian Influenza, H5N1, is the highly pathogenic influenza A virus subtype that is an emerging avian influenza virus that is causing global concern as a potential pandemic threat. It is often referred to simply as "bird flu" or "avian influenza", even though it is only one of many subtypes. Since the first human H5N1 outbreak occurred in 1997, there has been an increasing number of HPAI H5N1 bird-to-human transmissions, leading to clinically severe and fatal human infections.

Also described herein are methods that comprise the administration of an antibacterial tetracycline compound to a subject, in need thereof, in an amount which is effective for the treatment of viral infections that cause respiratory illnesses (e.g., illnesses caused by CoV, Swine Influenza and Avian Influenza).

In the present invention, the treatment of the illnesses/diseases in a subject includes the prevention or attenuation of the symptoms and/or morbidity associated with the illnesses/diseases. Subjects include humans and all non-human animals, such as, for example, dogs, pigs, cats, cows, sheep and birds.

In one embodiment, in the present invention, the tetracycline compound is administered prophylactically to an individual who is at risk of developing COVID-19 (i.e., a subject in need thereof). Individuals who are at risk have one or more of the following characteristics:
- are elderly (e.g., over age 65 years old);
- are under stress and/or are depressed;
- have weakened immune systems or are immunocompromised;
- have been in contact with a person having, or suspected of having, the disease.

Individuals who have weakened immune systems include, for example, cancer patients, in particular those who are undergoing chemotherapy; individuals with transplants; individuals who take pharmaceuticals to suppress their immune response (e.g., corticosteroids, cyclosporine); individuals with **HIV** or AIDS; diabetics; individuals recovering from an illness, surgery or trauma; and individuals with poor nutrition.

Prophylactic administration of the tetracycline compound is especially suited for individuals who are at risk of developing one of the aforementioned illnesses/diseases (e.g., viral illnesses/diseases). Prophylactic administration of the tetracycline compound is also especially suited for individuals who are at risk of contracting one of the aforementioned diseases and cannot tolerate (or cannot benefit from) a vaccine. For example, it is known that many elderly individuals do not react efficiently to vaccines.

Prophylactic administration of the tetracycline compound is administration before the onset of aforementioned illnesses/diseases. For example, administration is at least a few days before the onset of one of the aforementioned diseases, at least a week before the onset of the disease, at least two weeks before the onset of the disease, at least a month before the onset of the disease, at least six months before the onset of the disease.

**In** another embodiment, the tetracycline compound is administered as soon it is first suspected that the individual has one of the aforementioned illnesses/diseases (i.e., a subject in need thereof). The earliest symptoms include respiratory symptoms, fever, cough, shortness of breath and breathing difficulties. The treatment can be continued for the duration of the disease.

### Tetracycline Compounds

The tetracycline compound is a non-antibacterial compound. The tetracyclines are a class of compounds of which tetracycline is the parent compound. Tetracycline has the following general structure:

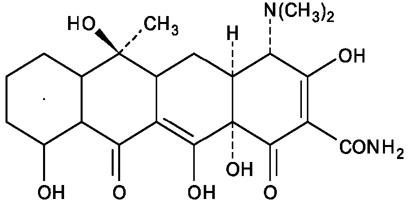

The numbering system of the multiple ring nucleus is as follows:

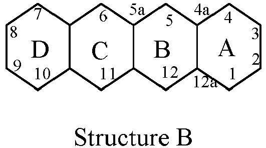

Tetracycline, as well as the 5-hydroxy (oxytetracycline, e.g., Terramycin) and 7-chloro (chlorotetracycline, e.g., Aureomycin) derivatives, exist in nature, and are all well known antibacterials. Semisynthetic derivatives such as 7-dimethylaminotetracycline (minocycline) and 6α-deoxy-5-hydroxytetracycline (doxycycline) are also known tetracycline antibacterials. Natural tetracyclines may be modified without losing their antibacterial properties, although certain elements of the structure must be retained to do so.

WO 2005/082860 describes the use of tetracyclines as calpain inhibitors, particularly inhibitors of calpain I and II.

Some examples of antibacterial *(i.e.,* antimicrobial) tetracycline compounds include doxycycline, minocycline, tetracycline, oxytetracycline, chlortetracycline, demeclocycline, lymecycline and their pharmaceutically acceptable salts.

Non-antibacterial tetracycline compounds are structurally related to the antibacterial tetracyclines, but have had their antibacterial activity substantially or completely eliminated by chemical modification. For example, non-antibacterial tetracycline compounds are at least about ten times, preferably at least about twenty five times, less antibacterial than doxycycline. In other words, non-antibacterial tetracycline compounds are incapable of achieving antibacterial activity comparable to that of doxycyline at concentrations at least about ten times, preferably at least about twenty five times, greater than that of doxycycline.

Examples of chemically modified non-antibacterial tetracyclines include 4-de(dimethylamino)tetracycline (CMT-1), tetracyclinonitrile (CMT-2), 6-demethyl-6-deoxy-4-de(dimethylamino)tetracycline (CMT-3), 7-chloro-4-de(dimethylamino)-tetracycline (CMT-4), tetracycline pyrazole (CMT-5), 4-hydroxy-4-de(dimethylamino)-tetracycline (CMT-6), 4-de(dimethylamino-12α-deoxytetracycline (CMT-7), 6-deoxy-5α-hydroxy-4-de(dimethylamino)tetracycline (CMT-8), 4-de(dimethylamino)-12α-deoxyanhydrotetracycline (CMT-9), and 4-de(dimethylamino)minocycline (CMT-10).

Throughout this specification, parameters are defined by maximum and minimum amounts. Each minimum amount can be combined with each maximum amount to define a range.

According to the present invention, a tetracycline compound is administered in a sub-antibacterial amount. A sub-antibacterial amount of a tetracycline compound is any amount that results in a tetracycline plasma concentration: (i) which is effective to treat COVID-19 but (ii) which has no, or substantially no, antibacterial activity.

A treatment is effective if it causes one or more of: a reduction/inhibition/prevention of any symptom associated with one of the aforementioned diseases, especially, a reduction/inhibition/prevention of the breathlessness and lung ailments; and/or shortening of the duration of an episode of such disease.

A concentration of a tetracycline compound having substantially no antibacterial activity is any concentration that does not significantly prevent the growth of bacteria. That is, a microbiologist would not consider the growth of bacteria to be inhibited from a clinical point of view.

One way in which to quantify the antibacterial activities of tetracyclines is by a measure called minimum inhibitory concentration (MIC), as is known by a skilled artisan.

An MIC is the minimum tetracycline concentration that inhibits the growth of a particular strain of bacteria *in vitro.* MIC values are determined using standard procedures. Standard procedures are, for example, based on a dilution method (broth or agar), or an equivalent, using standard concentrations of inoculum and tetracycline powder. See, for example, National Committee for Clinical Laboratory Standards. Performance Standards for Antimicrobial Susceptibility Testing - Eleventh Informational Supplement. NCCLS Document M100-S11, Vol. 21, No. 1, NCCLS, Wayne, PA, January, 2001.

In order to inhibit the growth of a strain of bacteria *in vivo,* a tetracycline compound achieves a plasma concentration in excess of the MIC for the strain. Plasma concentration refers to the concentration of a tetracycline compound measured in an individual's blood sample taken at steady state. Steady state is generally achieved after dosing for five to seven terminal half-lives. The half-lives of different tetracycline compounds vary from hours to days.

In the present invention, a tetracycline compound is administered in an amount that is effective, as described above, and that results in a plasma concentration which is significantly below the MIC for commonly-occurring bacteria. Such amounts are considered to have no, or substantially no, antibacterial activity. Examples of commonly-occurring bacteria that are susceptible to tetracyclines are *Escherichia coli* (*e.g.,* ATCC 25922 and 25922); *Neisseria gonorrhoeae* (*e.g.,* ATCC 49226); *Staphylococcus aureus* (*e.g.,* ATCC 29213 and 25213); and *Streptococcus pneumoniae* (*e.g.,* ATCC 49619).

For example, in the present invention, a tetracycline compound is administered in an amount that results in a plasma concentration which is less than approximately 80%, 75%, 70%, 65%, 60%, 55%, 50%, 45%, 40%, 35%, 30%, 25%, 20%, 15%, 10%, 5%, 1% or 0.5% of the MIC for the commonly-occurring bacteria mentioned above. A skilled artisan can readily determine the amount of a particular tetracycline compound to administer to achieve such concentrations.

The sub-antibacterial amount of an antibacterial tetracycline compound can also be expressed by daily dose. The daily dose of an antibacterial tetracycline compound is any amount that is sufficient to produce the effective, sub-antibacterial plasma concentrations described above. Such dose can, for example, be expressed as a percentage of a minimum antibacterial daily dose.

A skilled artisan knows, or is able routinely to determine, the minimum antibacterial daily dose for tetracycline compounds. Examples of suitable sub-antibacterial doses of antibacterial tetracycline compounds for the treatments described in the present specification include less than approximately: 80%, 75%, 70%, 65%, 60%, 55%, 50%, 45%, 40%, 35%, 30%, 25%, 20%, 15%, 10%, 5%, 1% and 0.5% of a minimum antibacterial dose.

There is no necessary minimum effective amount of the tetracycline compound, as long as the amount administered is capable of providing the effective treatment. For example, when the amount is expressed as a percentage of the MIC plasma concentration, suitable minimum plasma concentrations include approximately 0.1%, 0.5%, 0.8% and 1% of the MIC plasma concentration. When the amount is expressed as a minimum actual plasma concentration, suitable actual plasma concentrations include approximately 0.01 µg/ml, 0.05 µg/ml, 0.1 µg/ml, 0.15 µg/ml, 0.2 µg/ml, 0.25 µg/ml and 0.3 µg/ml. When the dose is expressed as a percentage of a minimum antibacterial daily dose, the percentage is approximately 0.1%, 0.2%, 0.5%, 1%, 1.5% and 2% of the minimum antibacterial dose.

The tetracycline compound is a non-antibacterial tetracycline compound, which is CMT-3 discussed above. Since CMTs have no, or substantially no, antibacterial activity, they can be administered at any effective dose at which side effects, if any, are acceptable. There is no risk of indiscriminate killing of bacteria, and the resulting threat of developing resistant bacteria.

The CMT is 6-demethyl-6-deoxy-4-de(dimethylamino)tetracycline (CMT-3). CMT-3 is administered in doses of up to about 200 mg/day, preferably about 150 mg/day, more preferably about 100 mg/day, or in amounts that result in plasma concentrations of up to about 50 µg/ml, about 40 µg/ml or about 30 µg/ml. For example, a dose of about 10 to about 20 mg/day of CMT-3 produces plasma concentrations in humans of about 1.0 µg/ml. In one embodiment, CMT-3 is administered in a dose of 40-50mg/ day.

There is no necessary minimum effective dose of CMTs. Some typical minimum plasma concentrations of CMTs include, for example, about 0.01 µg/ml, 0.1 µg/ml, 0.8 µg/ml, and 1.0 µg/ml. Some typical minimum daily doses of CMTs include about 0.05 mg/day, about 0.1 mg/day, about 0.5 mg/day, about 1 mg/day, about 5 mg/day, or about 10 mg/day.

In a preferred embodiment, CMT-3 is administered by controlled release over a 24 hour period.

In another embodiment, the tetracycline compound is administered as a pharmaceutical composition comprising an active ingredient wherein the active ingredient consists essentially of a tetracycline compound in an amount that is effective to treat the conditions described in the present specification, but has substantially no antibacterial activity.

The actual preferred amounts of tetracycline compounds in a specified case will vary according to the particular compositions formulated, the mode of application, the particular sites of application, and the subject being treated (*e.g.,* age, gender, size, tolerance to drug, species, etc.)

The tetracycline compound can be in the form of pharmaceutically acceptable salts of the compounds. The term "pharmaceutically acceptable salt" refers to a salt prepared from a well-tolerated, nontoxic tetracycline compound and an acid or base. The acids may be inorganic or organic acids of tetracycline compounds. Examples of inorganic acids include hydrochloric, hydrobromic, nitric hydroiodic, sulfuric, and phosphoric acids. Examples of organic acids include carboxylic and sulfonic acids. The radical of the organic acids may be aliphatic or aromatic. Some examples of organic acids include formic, acetic, phenylacetic, propionic, succinic, glycolic, glucuronic, maleic, furoic, glutamic, benzoic, anthranilic, salicylic, phenylacetic, mandelic, embonic (pamoic), methanesulfonic, ethanesulfonic, panthenoic, benzenesulfonic, stearic, sulfanilic, alginic, tartaric, citric, gluconic, gulonic, arylsulfonic, and galacturonic acids. Appropriate organic bases may be selected, for example, from N,N-dibenzylethylenediamine, chloroprocaine, choline, diethanolamine, ethylenediamine, meglumine (N-methylglucamine), and procaine.

The tetracycline compound of the invention can be administered without administering a bisphosphonate compound. Bisphosphonates compounds are related to inorganic pyrophosphonic acid. The bisphosphonates include, as non-limiting examples, alendronate (4-amino-1-hydroxybutylidene bisphosphonic acid), clodronate (dichloromethane diphosphonic acid), etidronate (1-hydroxyethylidene diphosphanic acid) and pamidronate (3-amino-1-hydroxypropylidene bisphosphonic acid); also risedronate ([2-(3-pyridinyl)ethylidene] hydroxy bisphosphonic acid), tiludronate, *i.e.,* tiludronic acid (4-chlorophenylthiomethylene bisphosphonic acid) and zolendronate.

Preferably, the tetracycline compound has low phototoxicity, or is administered in an amount that results in a plasma level at which the phototoxicity is acceptable. Phototoxicity is a chemically-induced photosensitivity. Such photosensitivity renders skin susceptible to damage, e.g., sunburn, blisters, accelerated aging, erythemas and eczematoid lesions, upon exposure to light, in particular ultraviolet light. The preferred amount of the tetracycline compound produces no more phototoxicity than is produced by the administration of a 40 mg total daily dose of doxycycline.

Examples of tetracycline compounds with low phototoxicity include, but are not limited to, tetracycline compounds having general formulae: 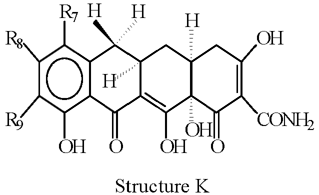 wherein: R7, R8, and R9 taken together in each case, have the following meanings:

| R7 | R8 | R9 | |
|---|---|---|---|
| hydrogen | hydrogen | amino | (CMT-308) |
| hydrogen | hydrogen | palmitamide | (CMT-311) |
| hydrogen | hydrogen | dimethylamino | (CMT-306) |

and 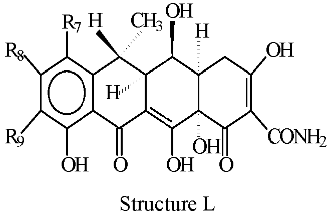 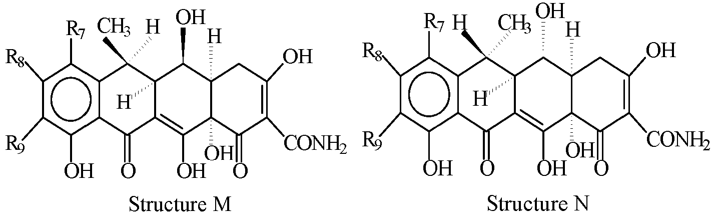 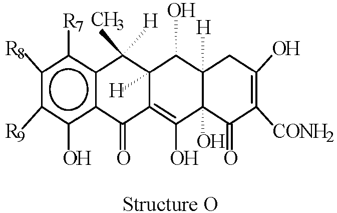 wherein: R7, R8, and R9 taken together in each case, have the following meanings:

| R7 | R8 | R9 | |
|---|---|---|---|
| hydrogen | hydrogen | acetamido | (CMT-801) |
| hydrogen | hydrogen | dimethylaminoacetamido | (CMT-802) |
| hydrogen | hydrogen | palmitamide | (CMT-803) |
| hydrogen | hydrogen | nitro | (CMT-804) |
| hydrogen | hydrogen | amino | (CMT-805) |

and 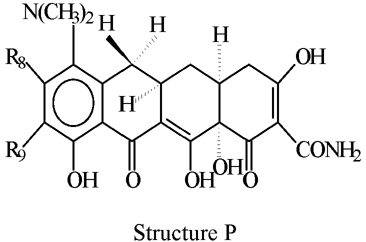 wherein: R8, and R9 taken together are, respectively, hydrogen and nitro (CMT-1002).

The tetracycline compound is administered systemically. For the purposes of this specification, "systemic administration" means administration to a human by a method that causes the compounds to be absorbed into the bloodstream.

Preferably, the tetracycline compound is administered orally by any method known in the art. For example, tetracyclines can be administered in the form of tablets, capsules, pills, troches, elixirs, suspensions, syrups, wafers, chewing gum and the like.

Additionally, the tetracycline compound can be administered enterally or parenterally, e.g., intravenously; intramuscularly; subcutaneously, as injectable solutions or suspensions; intraperitoneally; or rectally. Administration can also be intranasally, in the form of, for example, an intranasal spray; or transdermally, in the form of, for example, a patch.

For the pharmaceutical purposes described above, the tetracycline compound of the invention can be formulated per se in pharmaceutical preparations, optionally, with a suitable pharmaceutical carrier (vehicle) or excipient, as understood by practitioners in the art. These preparations can be made according to conventional chemical methods.

In the case of tablets for oral use, carriers commonly used include lactose and corn starch, and lubricating agents such as magnesium stearate are commonly added. For oral administration in capsule form, useful carriers include lactose and corn starch. Further examples of carriers and excipients include milk, sugar, certain types of clay, gelatin, stearic acid or salts thereof, calcium stearate, talc, vegetable fats or oils, gums and glycols.

When aqueous suspensions are used for oral administration, emulsifying and/or suspending agents are commonly added. In addition, sweetening and/or flavoring agents may be added to the oral compositions.

For intramuscular, intraperitoneal, subcutaneous and intravenous use, sterile solutions of the tetracycline compounds can be employed, and the pH of the solutions can be suitably adjusted and buffered. For intravenous use, the total concentration of the solute(s) can be controlled in order to render the preparation isotonic.

The tetracycline compound of the present invention can further comprise one or more pharmaceutically acceptable additional ingredient(s) such as alum, stabilizers, buffers, coloring agents, flavoring agents, and the like.

The tetracycline compound may be administered at intervals. For example, the tetracycline compound may be administered 1-6 times a day, preferably 1-4 times a day.

The tetracycline compound may be administered by controlled release. Controlled release administration is a method of drug delivery to achieve a certain level of the drug over a particular period of time. The level typically is measured by plasma concentration. Methods for controlled release of drugs are well known in the art. Further description of methods of delivering tetracycline compounds by controlled release can be found in international patent application PCT/US02/10748 (assigned to Galderma Laboratories, L.P.).

The tetracycline compounds are prepared by methods known in the art. For example, natural tetracyclines may be modified without losing their antibacterial properties, although certain elements of the structure must be retained. The modifications that may and may not be made to the basic tetracycline structure have been reviewed by Mitscher in The Chemistry of Tetracyclines, Chapter 6, Marcel Dekker, Publishers, New York (1978). According to Mitscher, the substituents at positions 5-9 of the tetracycline ring system may be modified without the complete loss of antibacterial properties. Changes to the basic ring system or replacement of the substituents at positions 1-4 and 10-12, however, generally lead to synthetic tetracyclines with substantially less or effectively no antibacterial activity.

Preferably, the subject (e.g., human) is monitored during treatment. Monitoring is accomplished by observing a positive treatment result. After a positive result is observed, treatment is continued.

### CHEMICAL NAMES OF THE CMT COMPOUNDS

| | |
|---|---|
| CMT-1 | 4-dedimethylaminotetracycline |
| CMT-3 | 6-demethyl-6-deoxy-4-dedimethylaminotetracycline |
| CMT-301 | 7-bromo-6-demethyl-6-deoxy-4-dedimethylaminotetracycline |
| CMT-302 | 7-nitro-6-demethyl-6-deoxy-4-dedimethylaminotetracycline |
| CMT-303 | 9-nitro-6-demethyl-6-deoxy-4-dedimethylaminotetracycline |
| CMT-304 | 7-acetamido-6-demethyl-6-deoxy-4-dedimethylaminotetracycline |
| CMT-305 | 9-acetamido-6-demethyl-6-deoxy-4-dedimethylaminotetracycline |
| CMT-306 | 9-dimethylamino-6-demethyl-6-deoxy-4-dedimethylaminotetracycline |
| CMT-307 | 7-amino-6-demethyl-6-deoxy-4-dedimethylaminotetracycline |
| CMT-308 | 9-amino-6-demethyl-6-deoxy-4-dedimethylaminotetracycline |
| CMT-309 | 9-dimethylaminoacetamido-6-demethyl-6-deoxy-4-dedimethylaminotetracycline |
| CMT-310 | 7-dimethylamino-6-demethyl-6-deoxy-4-dedimethylaminotetracycline |
| CMT-311 | 9-palmitamide-6-demethyl-6-deoxy-4-dedimethylaminotetracycline |
| CMT-312 | 2-CONHCH₂-pyrrolidin-1-yl-6-demethyl-6-deoxy-4-dedimethylaminotetracycline |
| CMT-313 | 2-CONHCH₂-piperidin-1-yl-6-demethyl-6-deoxy-4-dedimethylaminotetracycline |
| CMT-314 | 2-CONHCH₂-morpholin-1-yl-6-demethyl-6-deoxy-4-dedimethylaminotetracycline |
| CMT-315 | 2-CONHCH₂-piperazin-1-yl-6-demethyl-6-deoxy-4-dedimethylaminotetracycline |
| CMT-4 | 7-chloro-4-dedimethylaminotetracycline |
| CMT-5 | tetracycline pyrazole |
| CMT-6 | 4-hydroxy-4-dedimethylaminotetracycline |
| CMT-7 | 4-dedimethylamino-12α-deoxytetracycline |
| CMT-8 | 4-dedimethylaminodoxycycline |
| CMT-801 | 9-acetamido-4-dedimethylaminodoxycycline |
| CMT-802 | 9-dimethylaminoacetamido-4-dedimethylaminodoxycycline |
| CMT-803 | 9-palmitamide-4-dedimethylaminodoxycycline |
| CMT-804 | 9-nitro-4-dedimethylaminodoxycycline |
| CMT-805 | 9-amino-4-dedimethylaminodoxycycline |
| CMT-806 | 9-dimethylamino-4-dedimethylaminodoxycycline |
| CMT-807 | 2-CONHCH₂-pyrrolidin-1-yl-4-dedimethylaminodoxycycline |
| CMT-808 | 2-CONHCH₂-piperidin-1-yl-4-dedimethylaminodoxycycline |
| CMT-809 | 2-CONHCH₂-piperazin-1-yl-4-dedimethylaminodoxycycline |
| CMT-10 | 4-dedimethylaminominocycline (a.k.a. CMT-310) |
| CMT-1001 | 7-trimethylammonium-4-dedimethylaminosancycline |
| CMT-1002 | 9-nitro-4-dedimethylaminominocycline |

## Claims

1. A non-antibacterial tetracycline compound in an amount that is effective to treat COVID-19 but has no antibacterial activity, for use in a method of treating COVID-19, in a subject in need thereof, wherein the non-antibacterial tetracycline compound is 6-demethyl-6-deoxy-4-de(dimethylamino)tetracycline (CMT-3), and wherein the method comprises the systemic administration of the non-antibacterial tetracycline compound.

2. A non-antibacterial tetracycline compound for use according to Claim 1, wherein treatment is begun before the onset of COVID-19.

## Patentansprüche

1. Nicht-antibakterielle Tetracyclinverbindung in einer Menge, die wirksam zur Behandlung von COVID-19 ist, aber keine antibakterielle Aktivität hat, zur Verwendung in einem Verfahren zur Behandlung von COVID-19 bei einem Patienten, der dies benötigt, wobei die nicht-antibakterielle Tetracyclinverbindung 6-Demethyl-6-desoxy-4-de(dimethylamin)tetracyclin (CMT-3) ist, und wobei das Verfahren die systemische Verabreichung der nicht-antibakteriellen Tetracyclinverbindung umfasst.

2. Nicht-antibakterielle Tetracyclinverbindung zur Verwendung nach Anspruch 1, wobei die Behandlung vor dem Ausbruch von COVID-19 begonnen wird.

## Revendications

1. Composé de tétracycline non antibactérien en une quantité qui est efficace pour traiter la COVID-19 mais qui n'a pas d'activité antibactérienne, destiné à être utilisé dans un procédé de traitement de la COVID-19, chez un sujet qui en a besoin, où le composé de tétracycline non antibactérien est la 6-déméthyl-6-désoxy-4-dé(diméthylamino)-tétracycline (CMT-3), et où le procédé comprend l'administration systémique du composé de tétracycline non antibactérien.

2. Composé de tétracycline non antibactérien destiné à être utilisé selon la revendication 1, où le traitement est initié avant le déclenchement de la COVID-19.
